# EUROPEAN PATENT APPLICATION

(11) **EP 0 544 259 A1**
(43) Date of publication of application: **02.06.1993**
(21) Application number: 92120096.0
(22) Date of filing: 25.11.1992
(51) Int. Cl.: C08B 37/00, C08B 5/08, A61L 27/00, A61L 15/28

(54) **Water insoluble biocompatible hyaluronic and polyion complex and method of making the same**

(30) Priority: 27.11.1991 JP 312236/91
(71) Applicant: Lignyte Co., Ltd., Osaka (JP)
(72) Inventor: Uragami, Tadashi, Mino-shi, Osaka (JP); Tanaka, Yoshiaki, Osaka (JP); Nishida, Shinji, Sakai-shi, Osaka (JP)
(74) Representative: Goddar, Heinz J., Dr.

(57) **Abstract**

A water insoluble biocompatible hyaluronic acid polyion complex comprises hyaluronic acid and at least one biocompatible high molecular compound having amino or imino groups which are ionic bonded to carboxyl groups of hyaluronic acid. The polyion complex is made by reacting an alkalimetal salt of hyaluronic acid with the high molecular compound in an organic acid aqueous solution. The high molecular compound includes at least one selected from an amino polysaccharide such as chitosan, polyaminogalactosamine and triethanolamine alginate, etc., and a protein such as gelatine, casein, keratin, collagen, myosin and fibroin, etc. The hyaluronic acid polyion complex may be used in such in vivo applications as in artificial internal organs, artificial blood vessel and skin, etc.

## Description

### TECHNICAL FIELD

The present invention is directed to a water insoluble biocompatible hyaluronic acid polyion complex comprising hyaluronic acid and at least one biocompatible high molecular compound having amino or imino groups which are ionic bonded to carboxyl groups of hyaluronic acid, and a method of making the same.

### BACKGROUND ART

Hyaluronic acid which is represented by the following structural formula [1] is a kind of natural high molecular polysaccharide, and included in, for example, a vitreous body, an umbilical cord, a joint humor and comb of chicken, etc. 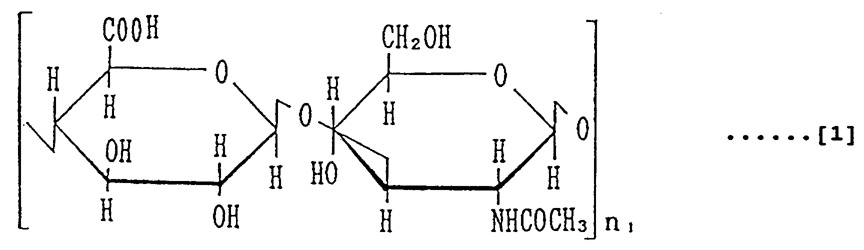
**n₁ : A positive number**
As hyaluronic acid has excellent water absorptivity, it is used in cosmetic applications to moisturize into skin. Hyaluronic acid also has excellent biocompatibility. It is expected that hyaluronic acid is used in such in vivo applications as in artificial blood vessel and artificial skin. However, since hyaluronic acid readily solves to water, it is so difficult to use hyaluronic acid for transplanting in a human body.

Japanese patent early publication [KOKAI] No. 1-124465 describes about a mucopolysaccharide incorporated with collagen and/or gelatine. Japanese patent early publication [KOKAI] No. 1-265970 describes about an aqueous solution including collagen and hyaluronic acid in case of using a water soluble collagen, and a collagen dispersed aqueous solution including hyaluronic acid in case of using a water insoluble collagen. Japanese patent early publication [KOKAI] No. 1-303151 describes that a composite consisting of N-acylchitosan and collagen is mixed with hyaluronic acid, so that thus mixed composite has water absorptivity. Japanese patent early publication [KOKAI] No. 63-300770 describes about a hyaluronic acid fixed protein film. That is, a protein film such as collagen, gelatine, and albumen, etc., is dipped into a hyaluronic acid aqueous solution, and then a water soluble carbodiimide reagent, or a cross-linking agent such as cyanuric chloride, etc., is added to the aqueous solution, so that the hyaluronic acid fixed protein film is formed. The fixed protein film has excellent water absorptivity. However, the products of the prior arts described above are not insolubilized to water.

On the other hand, UK Patent Application GB 2 151 244A describes that water insoluble, biocompatible hyaluronic acid preparations are made by subjecting hyaluronic acid to treatment with a cross-linking agent selected from formaldehyde, dimethylolurea, dimethylolethylene urea, ethylene oxide, a polyaziridine, a polyisocyanate and divinyl sulphone. Japanese Patent Publication [KOKOKU] No. 63-44383 describes about a water insoluble hyaluronic acid preparation for an antithrombus material. That is, a salt of hyaluronic acid is mixed with collagen or a compound of gelatine and collagen. And then, an article of a high molecular material is coated with the resulting mixture thereof. Thus coated article is treated with a cross-linking agent of a polyaldehyde such as glutaraldehyde and dialdehyde starch, so that the water insoluble hyaluronic acid preparation is obtained. However, as the cross-linking agents used in the prior arts do not have biocompatibility, there is a problem for using the products made according to the prior arts described above in the vivo applications in case that the cross-linking agents is left in the products. And also, the products of the prior arts may be a poor elasticity and softness occurring from bridge structures thereof.

The present invention relates to a water insoluble biocompatible hyaluronic acid polyion complex comprising hyaluronic acid and at least one biocompatible high molecular compound having amino or imino groups which are ionic bonded to carboxyl groups of hyaluronic acid. The polyion complex is insolubilized to water while keeping excellent water absorptivity thereof. Moreover, since each of hyaluronic acid and the high molecular compound has excellent biocompatibility, the polyion complex may be used as a film and tube, etc., for an artificial internal organ such as an artificial heart, an artificial blood vessel and skin, etc.

It is, therefore, a primary object of the present invention to provide a water insoluble biocompatible hyaluronic acid polyion complex comprising hyaluronic acid and at least one biocompatible high molecular compound having amino or imino groups which are ionic bonded to carboxyl groups of hyaluronic acid.

For making the polyion complex of the present invention, an alkalimetal salt of hyaluronic acid is used. The alkalimetal salt of hyaluronic acid has anions of carboxyl groups. On the other hand, amino or imino groups of the high molecular compound are cationized by reacting the high molecular compound with an organic acid, so that the high molecular compound having cations of amino or imino groups is obtained. When the alkalimetal salt of hyaluronic acid is reacted with the high molecular compound in the presence of the organic acid, the anions of carboxyl groups of hyaluronic acid bond with the cations of amino or imino groups of the high molecular compound to form a bridge structure between hyaluronic acid and the high molecular compound. As a result, the polyion complex of the present invention is obtained.

It is another object of the present invention to provide a method of making a water insoluble biocompatible hyaluronic acid polyion complex which is made by reacting an alkalimetal salt of hyaluronic acid with a biocompatible high molecular compound having amino or imino groups in the presence of an organic acid.

In the polyion complex of the present invention, the biocompatible high molecular compound includes at least one selected from an amino polysaccharide such as chitosan, polyaminogalactosamine and triethanolamine alginate, etc., and a protein such as gelatine, casein, keratin, collagen, myosin and fibroin, etc. On the other hand, the organic acid is selected from the group consisting of formic acid, acetic acid, propionic acid and butyric acid, etc.

By the way, a tube of the polyion complex of the present invention is made by the following method. That is, an aqueous solution of the alkalimetal salt of hyaluronic acid is mixed with an organic acid aqueous solution of the biocompatible high molecular compound, so that an aqueous solution including the polyion complex is formed. The aqueous solution is coated on the peripheral surface of a rod, and then dried to form the tube of the polyion complex around the rod. The rod is readily removed from the tube.

Therefore, it is a further object of the present invention to provide a method of making a tube of a water insoluble biocompatible hyaluronic acid polyion complex comprising hyaluronic acid and at least one biocompatible high molecular compound having amino or imino groups which are ionic bonded to carboxyl groups of hyaluronic acid.

On the other hand, a porous body of the polyion complex of the present invention is formed by the following method. That is, the aqueous solution of the alkalimetal salt of hyaluronic acid is mixed with the organic acid aqueous solution of the biocompatible high molecular compound while agitating a resulting mixture thereof, so that a sol solution including the polyion complex is formed. The sol solution is dried by freeze-drying to obtain the porous body of the polyion complex.

Therefore, it is a still further object of the present invention to provide a method of making a porous body of a water insoluble biocompatible hyaluronic acid polyion complex comprising hyaluronic acid and at least one biocompatible high molecular compound having amino or imino groups which are ionic bonded to carboxyl groups of hyaluronic acid.

A fibre-sheet of the polyion complex of the present invention is formed by the following method. That is, a first aqueous solution of the alkalimetal salt of hyaluronic acid and a second organic acid aqueous solution of the biocompatible high molecular compound are prepared. one of the first and second solutions is introduced into the other solution through a nozzle, so that an aqueous solution including polyion complex fibers are obtained. The polyion complex fibers are removed from the aqueous solution to form a wet sheet of the polyion complex fibers, and then the wet sheet is dried to obtain the fibre-sheet of the polyion complex.

Therefore, it is a further object of the present invention to provide a method of making a fibre-sheet of a water insoluble biocompatible hyaluronic acid polyion complex comprising hyaluronic acid and at least one biocompatible high molecular compound having amino or imino groups which are ionic bonded to carboxyl groups of hyaluronic acid.

The water insoluble biocompatible hyaluronic acid polyion complex of the present invention and method of making the polyion complex will be detailed hereinafter.

### DETAILED DESCRIPTION OF THE INVENTION

Hyaluronic acid which is represented by the structural formula 1 is a kind of natural high molecular polysaccharide. A molecular weight of hyaluronic acid is about 1.5 million to 2.5 million. Hyaluronic acid is reacted with an alkalimetal hydroxide to form an alkalimetal salt of hyaluronic acid, as represented by the following structural formula [2]. 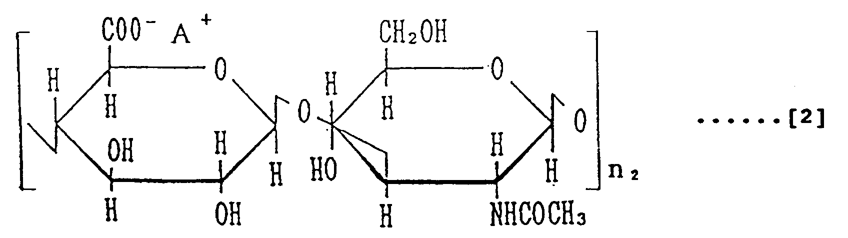
**A : Alkalimetal**
**n₂ : A positive number**
The alkalimetal is selected from the group of lithium, sodium, potassium and rubidium, etc. For example, hyaluronic acid is reacted with an enough amount of sodium hydroxide to form sodium hyaluronate. It is not concerned that sodium hyaluronate on the market is used in the present invention. On the other hand, a biocompatible high molecular compound having amino or imino groups includes at least one selected from an amino polysaccharide such as chitosan, polyaminogalactosamine and triethanolamine alginate, etc., and a protein such as gelatine, casein, keratin, collagen, myosin and fibroin, etc. The alkalimetal salt of hyaluronic acid is reacted with the high molecular compound in an organic acid aqueous solution to form a water insoluble biocompatible polyion complex. The polyion complex comprises hyaluronic acid and the high molecular compound having amino or imino groups which are ionic bonded to carboxyl groups of the hyaluronic acid. For example, chitosan and collagen is reacted with the alkalimetal salt of hyaluronic acid in the organic acid aqueous solution, so that a polyion complex consisting of chitosan, collagen and hyaluronic acid is obtained. On the other hand, as another method to make the polyion complex, after the alkalimetal salt of hyaluronic acid is reacted with collagen in the organic acid aqueous solution to form a polyion complex consisting of hyaluronic acid and collagen, the polyion complex is reacted with chitosan in the organic acid aqueous solution, so that the polyion complex consisting of chitosan, collagen and hyaluronic acid is obtained. Similarly, it is not concerned that chitosan and gelatine is reacted with the alkalimetal salt of hyaluronic acid in the organic acid aqueous solution, so that a polyion complex consisting of chitosan, gelatine and hyaluronic acid is obtained. The organic acid is selected from the group consisting of formic acid, acetic acid, propionic acid and butyric acid, etc. It is preferred that a mixture ratio of the alkalimetal salt of hyaluronic acid : the high molecular compound is in a 1 : 1 molar ratio. However, as necessary, it is not concerned that an excess of the alkalimetal salt of hyaluronic acid is mixed with the high molecular compound, or an excess of the high molecular compound is mixed with the alkalimetal salt of hyaluronic acid. By the way, the concentration of the organic acid aqueous solution is prepared to be higher than that necessary for dissolving the high molecular compound into the aqueous solution. For example, when a formic acid aqueous solution is used to dissolve the high molecular compound thereinto, it is preferred that the formic acid aqueous solution includes more than 20% by weight of formic acid.

For more understanding of the polyion complex of the present invention, a reaction of the alkalimetal salt of hyaluronic acid with chitosan of the high molecular compound in the organic acid aqueous solution is explained below. That is to say, carboxyl groups of hyaluronic acid are anionized by a reaction between hyaluronic acid and the alkalimetal hydroxide. Therefore, the alkalimetal salt of hyaluronic acid has anions of carboxyl groups, as represented by the structural formula 2. On the other hand, amino groups of chitosan are cationized by a reaction between chitosan and the organic acid, so that chitosan in the organic acid aqueous solution has cations of amino groups, as represented by the following structural formula [3]. 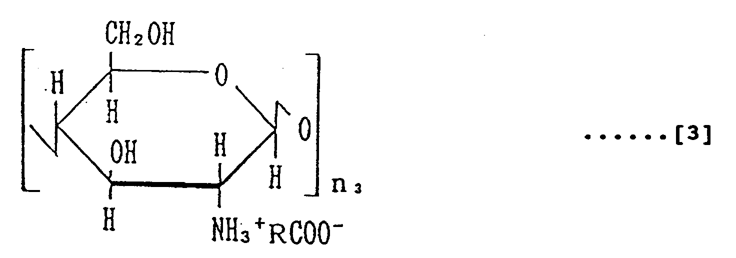
**R : H or C**_{**m**}**H**_{**m+1**} **(m : A positive number)**
**n₃ : A positive number**
Therefore, when the alkalimetal salt of hyaluronic acid is reacted with chitosan in the organic aqueous solution, the anions of carboxyl groups of hyaluronic acid bond with the cations of amino groups of chitosan to form a bridge structure between hyaluronic acid and chitosan. As a result, a water insoluble biocompatible polyion complex consisting of chitosan and hyaluronic acid is obtained, as represented by the following structural formula [4]. It is possible to form a cation or anion rich polyion complex by differing the number of repeating units of chitosan from that of hyaluronic acid. 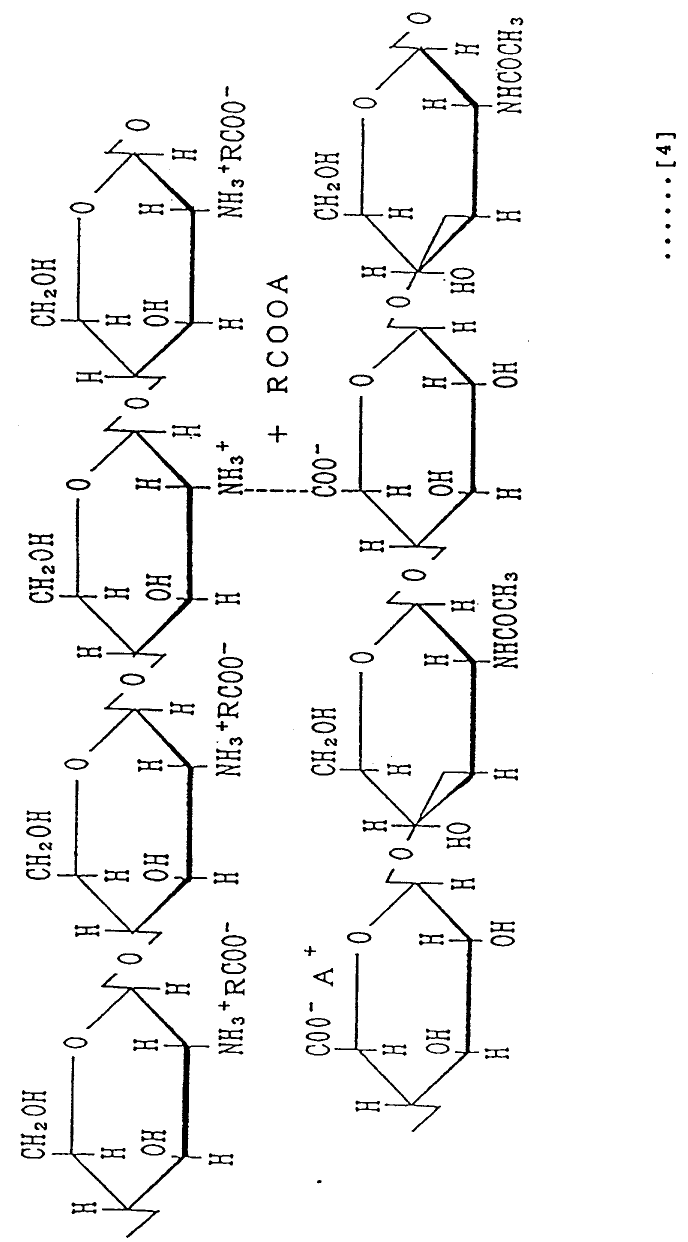
And then, an aqueous solution including the polyion complex is dried. For example, when the aqueous solution is cast into a desired mold, and then dried, it is so easy to form a desired shape of the polyion complex. On the other hand, when the aqueous solution is uniformly cast onto a onto a petri dish, and then dried, a polyion complex film is formed on the petri dish.

A method of forming a thin film of the polyion complex of the present invention is described below. A first aqueous solution of the alkalimetal salt of hyaluronic acid and a second organic acid aqueous solution of the high molecular compound are prepared. The first aqueous solution is uniformly onto the petri dish, and then the second aqueous solution is uniformly cast onto the first aqueous solution. Instantly the first aqueous solution is contacted with the second aqueous solution, the polyion complex is formed at the interface between the first and second aqueous solutions. Therefore, the thin film of the polyion complex is formed in such a manner of an interfacial polymerization. The thin film is washed with water to remove the first and second aqueous solutions left thereon, and then dried. On the other hand, it is not concerned that the thin film of the polyion complex is formed according to the following method. That is, the first aqueous solution is uniformly cast onto the petri dish, and then dried, so that a film of the alkalimetal salt of hyaluronic acid is formed on the petri dish. Instantly the second aqueous solution is uniformly cast onto the film, the thin film of the polyion complex is formed at the interface between the second aqueous solution and the film. The thin film of the polyion complex is washed with water to remove the film of the alkalimetal salt of hyaluronic acid left on a surface of the thin film, and also the second aqueous solution left on the other surface of the thin film, and then dried.

A method of forming a porous body of the polyion complex of the present invention is described below. The first aqueous solution of the alkalimetal salt of hyaluronic acid is mixed with the second organic acid aqueous solution of the high molecular compound while agitating a resulting mixture thereof by a mixer or a blender, etc., so that a sol solution including the polyion complex is obtained. As the sol solution has extremely high water absorptivity, water included in the sol solution does not vaporize for a long time period except when the sol solution is heated. When the agitation is not enough to form the sol solution uniformly dispersing the polyion complex, it is necessary to perform the agitation hard, for example, by accelerating a speed of the blender. The sol solution is dried by freeze-drying to obtain the porous body of the polyion complex. On the other hand, it is not concerned that the sol solution is cast into a desired mold, and then dried to obtain a desired shape of the polyion complex, and also the sol solution is uniformly cast onto the petri dish, and dried to obtain a polyion complex film.

A method of forming a polyion complex tube of the present invention is described below. The alkalimetal salt of hyaluronic acid and the high molecular compound are dissolved in the organic acid aqueous solution to form an aqueous solution including the polyion complex. Subsequently, a rod which is made of glass or Teflon, etc., is dipped into the aqueous solution including the polyion complex, drawn up from the aqueous solution, and then dried, so that a polyion complex film is formed on the peripheral surface of the rod. As necessary, by repeating the like procedure, the polyion complex films are successively formed around the rod to obtain the polyion complex film of increased thickness. The polyion complex tube is obtained by removing the rod from the polyion complex film formed rod. It is not concerned that the rod is dipped into the sol solution including the polyion complex to form the polyion complex tube.

One particular method of making the polyion complex tube of the present invention is described below. That is, the rod is firstly dipped into the first aqueous solution of the alkalimetal salt of hyaluronic acid, and then dried to coat a film of the alkalimetal salt of hyaluronic acid around the rod. Instantly thus coated rod is dipped into the second organic acid aqueous solution of the high molecular compound, a thin polyion complex film is formed at the interface between the coated rod and the second aqueous solution. As necessary, by repeating the like procedure, the thin polyion complex films were successively formed around the rod to obtain the polyion complex film of increased thickness. Consequently, the polyion complex tube is obtained by removing the rod from the polyion complex film formed rod. By the way, it is not concerned that the rod is firstly dipped into the second aqueous solution, and then dipped into the first aqueous solution to form the polyion complex film around the rod.

Method of forming polyion complex fibers of the present invention is described below. The second aqueous solution is introduced through a nozzle little by little into the first aqueous solution. Of course, it is not concerned that the first aqueous solution is introduced through the nozzle into the second aqueous solution. Instantly the second aqueous solution is contacted with the first aqueous solution, the polyion complex fibers are formed by a reaction of hyaluronic acid and the high molecular compound. That is to say, the polyion complex fibers are formed in such a manner of a wet spinning.

Method of forming a nonwoven fabric of the polyion complex of the present invention is described below. The second aqueous solution is introduced through the nozzle into the first aqueous solution while agitating the first aqueous solution to form an aqueous solution including short fibers of the polyion complex. Of course, it is not concerned that the first aqueous solution is introduced through the nozzle into the second aqueous solution while agitating the second aqueous solution. And then, the short fibers are removed from the aqueous solution, and dried. Subsequently, the short fibers are uniformly dispersed in water. The short fibers are removed from the short fibers dispersed water to form a wet sheet of the short fibers. The wet sheet is dried to obtain the nonwoven fabric of the polyion complex.

Therefore, the water insoluble biocompatible polyion complex of the present invention can be used in such in vivo applications as in an artificial blood vessel and skin, etc. For example, the film and the nonwoven fabric of the polyion complex is used as the artificial skin, the tube thereof is used as the artificial blood vessel, and also the filament thereof is used for suturing in operation. When the polyion complex is coated on the surface of an artificial internal organ such as an artificial heart, the artificial blood vessel, a catheter for a blood vessel, a heart-lung machine, a tube for an artificial liver and blood bypass tube, etc., which is made of nylon, polyester, polyethylene, polypropylene and polyurethane, etc., thus coated artificial internal organ has capable of preventing a generation of thrombus and improving biocompatibility. By the way, as the cation or anion polyion complex of the present invention can be readily formed, as described above, the anion or cation rich polyion complex is used as an ion-exchanger. On the other hand, the polyion complex of the present invention also has hydrophilicity. Therefore, the polyion complex film may be used as a separation film which has capable of separating water from a mixed solution of alcohol and water, a film for blood dialysis, a film for blood filtration and film for separation of plasma from blood, etc.

### Example 1

To 200cc of formic acid aqueous solution comprising 20% by weight of formic acid, 0.57g of chitosan(chitosan with a deacetylation of 80% manufactured by TUCHIYOSHI CO.,LTD., the same chitosan used in another Examples and Comparative Example 2.) and 1.43g of sodium hyaluronate of HYALURONIC ACID KYOWA-HP(trade name of sodium hyaluronate manufactured by KYOWA HAKKO KOGYO CO., LTD., the same sodium hyaluronate used in another Examples and Comparative Example 1.) were dissolved, and then agitated at 25°C for 24 hours, so that an aqueous solution including a polyion complex consisting of chitosan and hyaluronic acid was formed. The polyion complex is illustrated by the following structural formula [5]. A molar ratio of chitosan : sodium hyaluronate was 50 : 50. As thus formed aqueous solution includes a lot of bubbles resulting from the agitation thereof, ultrasonic wave was radiated to the aqueous solution for 10 min to exclude the bubbles. Subsequently, 20g of the aqueous solution was uniformly cast onto a petri dish having the diameter of 9cm, and then dried for 6 hours at 60°C. As a result, a polyion complex film having the thickness of about 15µm was obtained on the petri dish. The polyion complex film was readily took off form the petri dish.

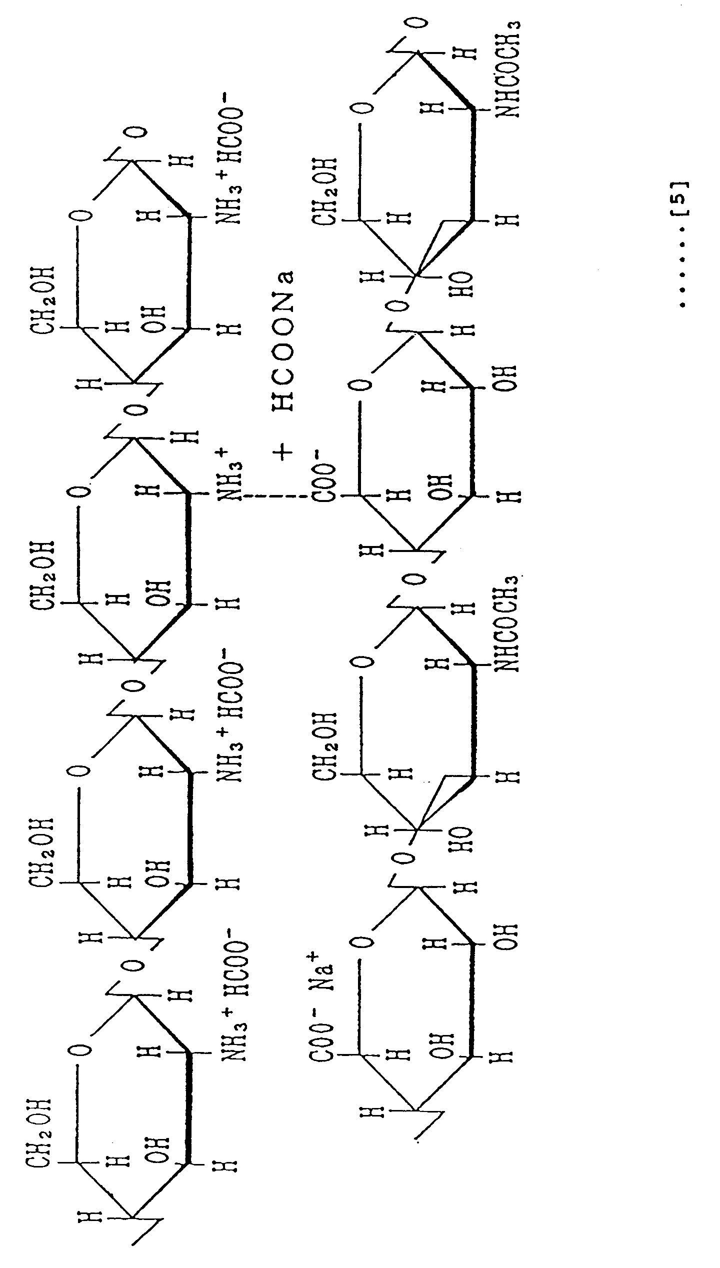

### Example 2

A polyion complex film of Example 2 was made according to the same method of Example 1 except that a molar ratio of chitosan : sodium hyaluronate was 30 : 70.

### Example 3

A polyion complex film of Example 3 was made according to the same method of Example 1 except that a molar ratio of chitosan : sodium hyaluronate was 70 : 30.

### Comparative Example 1

A hyaluronic acid film was made according to the same method of Example 1 except that 2.00g of sodium hyaluronate was dissolved to 200cc of the formic acid aqueous solution of Example 1 without adding chitosan. The thickness of the hyaluronic acid film is about 15µm.

### Comparative Example 2

A chitosan film was made according to the same method of Example 1 except that 2.00g of chitosan was dissolved to 200cc of the formic acid aqueous solution of Example 1 without adding hyaluronic acid. The thickness of the chitosan film is about 15µm.

Insolubility to water of each film of Examples 1, 2, 3, Comparative Examples 1 and 2, was examined by dipping the films into water which was kept at 25°C. The hyaluronic acid film of Comparative Example 1 and the chitosan film of Comparative Example 2 dissolved into water as soon as the films were dipped. On the other hand, there was no change with respect to the polyion complex films of the present invention, even after being dipped into water for a month. Therefore, it was confirmed that the polyion complex films have insolubility to water.

A degree of swelling of each film of Examples 1, 2, 3, Comparative Examples 1 and 2, was examined by hanging the films for three days in a vessel having a heated water at 40°C on the bottom of the vessel, or a vessel having a methanol aqueous solution comprising 10% by weight of methanol on the bottom of the vessel. The degree of swelling of each film was calculated by the following formula, that is, a degree of swelling = (A weight of a swelled film) / (A weight of a dried film). The swelled film was dried in a reduced pressure to measure a constant weight of the film. In case of hanging the films in the vessel having the heated water, the hyaluronic acid film and the chitosan film were melted down by exposing to moisture in the vessel. On the other hand, the polyion complex films of the present invention showed excellent water absorptivity without melting down thereof, as shown in TABLE 1. In case of hanging the films in the vessel having the methanol aqueous solution, the hyaluronic acid film and the chitosan film were not melted down by exposing to a vapor of the methanol aqueous solution for three days. As a result, the degree of swelling of each polyion complex film of Examples 1 and 2 was almost equal to that of the hyaluronic acid film or the chitosan film, as shown in TABLE 1.

**TABLE 1**

| A degree of swelling of each film of Examples 1, 2, 3, and Comparative Examples 1, 2. | | | |
|---|---|---|---|
| | Chitosan / Sodium hyaluronate (molar ratio) | Degree of swelling | |
| | | Water | Methanol aq. |
| Example 1 | 50/50 | 3.50 | 3.40 |
| Example 2 | 30/70 | 3.11 | 3.14 |
| Example 3 | 70/30 | 3.16 | 2.98 |
| Comparative Example 1 | 0/100 | --- | 3.30 |
| Comparative Example 2 | 100/0 | --- | 3.65 |

### Example 4

1.4g of sodium hyaluronate of Example 1 was dissolved in 200cc water while agitating a resulting mixture thereof, so that an aqueous solution **1** of sodium hyaluronate was obtained. As the aqueous solution **1** included a lot of bubbles resulting from the agitation, ultrasonic wave was radiated to the aqueous solution **1** to exclude the bubbles. On the other hand, 2.0g of chitosan of Example 1 was dissolved in 200cc of 1N acetic acid aqueous solution while agitating a resulting mixture thereof, so that an acetic acid aqueous solution **2** of chitosan was obtained. Similarly, ultrasonic wave was radiated to the aqueous solution **2** to exclude bubbles therefrom. The aqueous solution **1** was uniformly cast onto a square petri dish having the depth of 12mm and the side of 180mm, and then dried at 60°C for 6 hours, so that a sodium hyaluronate film was formed on the petri dish. The aqueous solution **2** was then uniformly cast onto the sodium hyaluronate film in the petri dish. The petri dish was sealed at its top opening by an aluminum foil, and kept at a room temperature for 12 hours. A polyion complex film consisting of hyaluronic acid and chitosan was formed at the interface between a surface of the sodium hyaluronate film and the aqueous solution **2**. Subsequently, the aqueous solution **2** left in the petri dish was removed, and also the sodium hyaluronate film was dissolved to water to separate from the polyion complex film. The polyion complex film was dried at 60°C for 6 hours. The thickness of the polyion complex film was about 20µm. The polyion complex film was dipped into water for a month to examine insolubility to water thereof. As a result, there was no change of the polyion complex film, even after being dipped into water for a month. Therefore, it was confirmed that the polyion complex film of Example 4 has insolubility to water.

### Example 5

To 100g of water, 50g of the aqueous solution **1** prepared in Example 4 and 50g of the aqueous solution **2** prepared in Example 4 were mixed, and agitated by a blender under the condition of 4000r.p.m. for 15 minutes, so that a sol solution including a polyion complex consisting of chitosan and hyaluronic acid was obtained. The sol solution had a viscosity of 13650cps which was measured by a synchronized motor viscosimeter under the condition of 12r.p.m. of rotor No.4 at 20°C in accordance with JISK-6909. Water included in the sol solution did not vaporize for a long time period except when the sol solution was heated. 150g of the sol solution was cast onto the square petri dish having the depth of 12mm and the side of 180mm, and kept for 24 hours at a room temperature to exclude bubbles resulting from the agitation form the sol solution. After 24 hours, the solo solution was dried at 60°C for 6 hours to form a film of the polyion complex on the petri dish. The polyion complex film was readily took off from the petri dish. Subsequently, the polyion complex film was washed three times in a methanol aqueous solution comprising 75% by weight of methanol, and then dried at 60°C for 2 hours. The thickness of the polyion complex film was about 8µm. The polyion complex film was dipped into water for a month to examine insolubility to water thereof. As a result, there was no change of the polyion complex film, even after being dipped into water for a month. Therefore, it was confirmed that the polyion complex film of Example 5 has insolubility to water.

### Example 6

To 200cc of 1N acetic acid aqueous solution, 6g of gelatine(manufactured by WAKO PURE CHEMICAL INDUSTRIES, LTD.) was dissolved, and agitated, so that an acetic acid aqueous solution **3** of gelatine was obtained, As the aqueous solution **3** included a lot of bubbles resulting from the agitation, ultrasonic wave was radiated to the aqueous solution **3** for 10 minutes to exclude the bubbles. A polyion complex film consisting of gelatine and hyaluronic acid of Example 6 was made according to the same method of Example 5 except that the aqueous solution **3** was used instead of the aqueous solution **2**. The thickness of the polyion complex film was about 10µm. The polyion complex film of Example 6 was dipped into water for a month to examine insolubility to water thereof. As a result, there was no change of the polyion complex film, even after being dipped into water for a month. Therefore, it was confirmed that the polyion complex film of Example 6 has insolubility to water.

### Example 7

To 1000cc of a formic acid aqueous solution comprising 20% by weight of formic acid, 7.2g of sodium hyaluronate of Example 1 and 2.9g of chitosan of Example 1 were dissolved, and agitated for 24 hours at a room temperature, so that an aqueous solution including a polyion complex consisting of chitosan and hyaluronic acid was obtained. 10cm length of a cylindrical glass rod having the diameter of 3mm was dipped into the aqueous solution including the polyion complex, and then drawn up from the aqueous solution. The glass rod adhering the aqueous solution was dried at 70°C for 10 minutes while being kept in horizontally and rotated around the axis of the glass rod so as not to drop the aqueous solution from the glass rod, and then cooled for 10 minutes by air, so that a thin polyion complex film was formed on the glass rod. By repeating the like procedure 50 times, the thin polyion complex films were successively formed around the glass rod to obtain a polyion complex film of increased thickness. A polyion complex tube of Example 7 was obtained by removing the glass rod from the polyion complex film in a methanol aqueous solution comprising 70% by weight of methanol. The polyion complex tube was then dried at 60°C for 1 hour. The polyion complex tube had the thickness of about 250µm and the inner diameter of about 2.6mm. The polyion complex tube was dipped into water for a month to examine insolubility to water thereof. As a result, there was no change of the polyion complex tube, even after being dipped into water for a month. Therefore, it was confirmed that the polyion complex tube of Example 7 has insolubility to water.

### Example 8

A polyion complex tube of Example 8 was made according to the same method of Example 7 except that the sol solution including the polyion complex prepared in Example 5 was used instead of the aqueous solution including the polyion complex of example 7. The polyion complex tube had the thickness of about 320µm and the inner diameter of 2.8mm. The polyion complex tube was dipped into water for a month to examine insolubility to water thereof. As a result, there was no change of the polyion complex tube, even after being dipping into water for a month. Therefore, it was confirmed that the polyion complex tube of Example 8 has insolubility to water.

### Example 9

10cm length of the cylindrical glass rod having the diameter of 3mm was dipped into the aqueous solution **1** of sodium hyaluronate of Example 4, and then drawn up from the aqueous solution **1**. The glass rod adhering the aqueous solution **1** was dried at 70°C for 10 minutes while being kept in horizontally and rotated around the axis of the glass rod so as not to drop the aqueous solution **1** from the glass rod, and then cooled for 10 minutes by air, so that a sodium hyaluronate film was formed around the glass rod. Subsequently, the glass rod was dipped into the acetic acid aqueous solution **2** of chitosan of Example 4 for 3 minutes to form a thin polyion complex film consisting of chitosan and hyaluronic acid at the interface between the sodium hyaluronate film and the aqueous solution **2**. The glass rod was drawn up from the aqueous solution **2**, and then hung in an atmosphere having a relative humidity of about 90% to remove an excess of the aqueous solution **2** left on the glass rod. The glass rod was dipped again into the aqueous solution **1** for 3 minutes to form a thin polyion complex film at the interface between the aqueous solution **2** left on the glass rod and the aqueous solution **1**. The glass rod was drawn up from the aqueous solution **1**, hung in the atmosphere having the relative humidity of about 90% to remove an excess of the aqueous solution **1** left on the glass rod, and then dried at 60°C for 1 hour, so that a fresh film of the polyion complex was integrally formed on the previous film of the polyion complex. By repeating the like procedure 50 times, the thin polyion complex films were successively formed around the glass rod to obtain a polyion complex film of increased thickness. Consequently, a polyion complex tube of Example 9 was obtained by removing the glass rod from the polyion complex film in the methanol aqueous solution comprising 70% by weight of methanol, and then dried at 60°C for 1 hour. The tube had the thickness of about 300µm and the inner diameter of about 2.5mm. The polyion complex tube was dipped into water for a month to examine insolubility to water thereof. As a result, there was no change of the polyion complex tube, even after being dipped into water for a month. Therefore, it was confirmed that the polyion complex tube of Example 9 has insolubility to water. By the way, in the method of making the polyion complex tube, it is not concerned that the glass rod is firstly dipped into the aqueous solution **2** to form a film of a chitosan salt of acetic acid around the glass rod, and then dipped into the aqueous solution **1** to form the polyion complex film around the glass rod. However, the polyion complex tube made according to the former method is better than that made according to the latter method.

### Example 10

1000cc of the aqueous solution **2** of Example 4 was put in a beaker having the volume of 2 liter. 600cc of the aqueous solution **1** of Example 4 was introduced through a nozzle having the inner diameter of 0.8mm little by little into the aqueous solution **2** while agitating the aqueous solution **2** by a agitator with 120r.p.m., so that an aqueous solution including short fibers made of a polyion complex consisting of chitosan and hyaluronic acid was obtained. After the aqueous solution including the short fibers was agitated for 12 hours, the short fibers were separated from the aqueous solution by a filtration, washed three times with water, washed two times with methanol, and then dried. 10g of the short fibers was uniformly dispersed into 100cc of water. The short fibers were removed from the short fibers dispersed water with a stainless wire cloth of 100 mesh, and then dried at 70°C for 1 hour, so that a nonwoven fabric of the polyion complex of Example 10 was obtained. The nonwoven fabric had the thickness of about 0.6mm and a porous structure capable of ventilating an air therethrough. The nonwoven fabric was dipped into water for a month to examine insolubility to water thereof. As a result, there was no change of the nonwoven fabric of the polyion complex, even after being dipped into water for a month. Therefore, it was confirmed that the nonwoven fabric of the polyion complex of Example 10 has insolubility to water.

### Example 11

25g of the sol solution including the polyion complex of Example 4 was cast onto a petri dish of Teflon having the diameter of 8mm and the depth of 12mm. The petri dish was sealed at its top opening by an aluminum foil, and then put for 12 hours in a freezer which is kept at -30°C. After removal of the freezer, thus freezed sol solution was placed in a vacuum chamber of freeze-dry available as "VD-80" from "Taitech Corp." to be dried without being defrosted under a reduced pressure of 0.01 Torr at a cold trap temperature of -80°C for 6 hours, so that a porous sheet of a polyion complex consisting of chitosan and hyaluronic acid was obtained. The porous sheet has 0.2mm thick. The porous sheet was dipped into water for a month to examine insolubility to water thereof. As a result, there was no change of the porous sheet, even after being dipped into water for a month. Therefore, it was confirmed that the porous sheet of the polyion complex of Example 11 has insolubility to water. And besides, the porous sheet has a spongelike structure which has extremely high water absorptivity.

The features disclosed in the foregoing description, in the claims and/or in the accompanying drawings may, both, separately and in any combination thereof, be material for realising the invention in diverse forms thereof.

## Claims

1. A water insoluble biocompatible hyaluronic acid polyion complex comprising hyaluronic acid and at least one biocompatible high molecular compound having amino or imino groups which are ionic bonded to carboxyl groups of hyaluronic acid.

2. A water insoluble biocompatible hyaluronic acid polyion complex as set forth in claim 1, wherein said biocompatible high molecular compound includes at least one selected from the group consisting of chitosan, polyaminogalactosamine, triethanolamine alginate, gelatine, casein, keratin, collagen, myosin and fibroin.

3. A water insoluble biocompatible hyaluronic acid polyion complex as set forth in claim 1, wherein said biocompatible high molecular compound is chitosan.

4. A water insoluble biocompatible hyaluronic acid polyion complex as set forth in claim 1, wherein said biocompatible high molecular compound essentially consists of chitosan and gelatine.

5. A water insoluble biocompatible hyaluronic acid polyion complex as set forth in claim 1, wherein said biocompatible high molecular compound essentially consists of chitosan and collagen.

6. A method of making a water insoluble biocompatible polyion complex comprising hyaluronic acid and at least one biocompatible high molecular compound having amino or imino groups which are ionic bonded to carboxyl groups of hyaluronic acid, said method comprising reacting an alkalimetal salt of hyaluronic acid with said biocompatible high molecular compound in the presence of an organic acid.

7. A method of making a water insoluble biocompatible hyaluronic acid polyion complex as set forth in claim 6, wherein said biocompatible high molecular compound includes at least one selected from the group consisting of chitosan, polyaminogalactosamine, triethanolamine alginate, gelatine, casein, keratin, collagen, myosin and fibroin.

8. A method of making a water insoluble biocompatible hyaluronic acid polyion complex as set forth in claim 6, wherein said organic acid is selected from the group consisting of formic acid, acetic acid, propionic acid and butyric acid.

9. A method of making a water insoluble biocompatible hyaluronic acid polyion complex as set forth in claim 6, wherein the alkalimetal salt of hyaluronic acid is reacted with chitosan in a formic acid aqueous solution.

10. A method of making a water insoluble biocompatible hyaluronic acid polyion complex as set forth in claim 6, wherein an aqueous solution of the alkalimetal salt of hyaluronic acid is contacted with an acetic acid aqueous solution of chitosan to form said polyion complex.

11. A method of making a water insoluble biocompatible hyaluronic acid polyion complex as set forth in claim 6, wherein an organic acid aqueous solution of chitosan is contacted with a solid body of a salt of hyaluronic acid to form said polyion complex.

12. A method of making a water insoluble biocompatible hyaluronic acid polyion complex as set forth in claim 6, wherein an aqueous solution of a salt of hyaluronic acid is contacted with a solid body of a chitosan salt of an organic acid to form said polyion complex.

13. A method of making a water insoluble biocompatible hyaluronic acid polyion complex as set forth in claim 6, wherein said method comprises the steps of:
mixing an aqueous solution of the alkalimetal salt of hyaluronic acid with an organic acid aqueous solution of said biocompatible high molecular compound while agitating a resulting mixture thereof to thereby a sol solution including the polyion complex formed by reaction of hyaluronic acid with said high molecular compound;
freeze-drying said sol solution to obtain a porous body of the polyion complex.

14. A method of making a water insoluble biocompatible hyaluronic acid polyion complex as set forth in claim 6, wherein said method comprises the steps of:
mixing an aqueous solution of the alkalimetal salt of hyaluronic acid with an organic acid aqueous solution of said high molecular compound to thereby prepare a solution containing the polyion complex formed by a reaction of hyaluronic acid with said high molecular compound;
coating on the peripheral surface of a rod with the resulting solution to form a coat around the rod;
drying the coat to form around the rod a tube of the polyion complex; and
removing the rod from the tube.

15. A method of making a water insoluble biocompatible hyaluronic acid polyion complex as set forth in claim 6, wherein said method comprises the steps of:
preparing a first aqueous solution of the alkalimetal salt of hyaluronic acid and a second organic acid aqueous solution of said biocompatible high molecular compound;
coating the peripheral surface of a rod with one of said first and second solutions to provide a resulting coat around the rod;
drying the coat;
coating the dried coat with the other solution to thereby effect reaction of forming a polyion complex film around the rod;
drying the resulting polyion complex film;
removing the rod from the resulting film to obtain a tube of the polyion complex film.

16. A method of making a water insoluble biocompatible hyaluronic acid polyion complex as set forth in claim 15, wherein the polyion complex films are successively formed around the rod by repeating the steps of coating a previously formed polyion complex film with the one solution to form an additional coat, drying said additional coat, coating the dried coat with the other solution to form an additional polyion complex film, and drying the additional polyion complex film.

17. A method of making a water insoluble biocompatible hyaluronic acid polyion complex as set forth in claim 6, wherein said method comprising the steps of:
mixing an aqueous solution of the alkalimetal salt of hyaluronic acid with an organic acid aqueous solution of said high molecular compound while agitating a resulting mixture thereof to thereby a sol solution including the polyion complex formed by
reaction of hyaluronic acid with said high molecular compound;
coating the periphery of a rod with the resulting sol solution to form a coat thereon;
drying the coat and removing the dried coat from the rod to obtain a tube of the polyion complex.

18. A method of making a water insoluble biocompatible hyaluronic acid polyion complex as set forth in claim 6, wherein said method comprising the steps of:
preparing a first aqueous solution of the alkali metal salt of hyaluronic acid and a second organic acid aqueous solution of said biocompatible high molecular compound;
introducing one of the first and second solution into the other solution through a nozzle to thereby form fibers of the polyion complex by a reaction of the hyaluronic acid and said high molecular compound,
removing the resulting polyion complex fibers from the solution;
forming a wet sheet of the polyion complex fibers; and
drying the wet sheet into a fibre-sheet of the polyion complex.

19. A method of making a water insoluble biocompatible hyaluronic acid polyion complex as set forth in claim 6, wherein said method comprising the steps of:
preparing a first aqueous solution of the alkali metal salt of hyaluronic acid and a second organic acid aqueous solution of said biocompatible high molecular compound;
introducing one of the first and second solution into the other solution through a nozzle to thereby form fibers of the polyion complex by a reaction of the hyaluronic acid and said high molecular compound,
removing the resulting polyion complex fibers from the solution and drying the same;
preparing an aqueous suspension of the polyion complex fibers;
forming a wet sheet of the polyion complex fibers from said suspension; and
drying the wet sheet into a fibre-sheet of the polyion complex.
